# FASCICULE DE BREVET EUROPEEN

(11) **EP 3 510 924 B1**
(45) Date de publication et mention de la délivrance du brevet: **30.12.2020**
(21) Numéro de dépôt: 18206041.8
(22) Date de dépôt: 13.11.2018
(51) Int. Cl.: A61B 5/083, A61M 16/00, A61M 16/04, A61M 16/08, A61H 31/00

(54) **APPAREIL DE VENTILATION POUR RÉANIMATION CARDIO-PULMONAIRE AVEC AFFICHAGE DE LA TENDANCE EN CO2**
BEATMUNGSGERÄT FÜR DIE HERZ-LUNGEN-WIEDERBELEBUNG MIT ANZEIGE DER CO2-TENDENZ
VENTILATION APPARATUS FOR CARDIOPULMONARY RESUSCITATION WITH DISPLAY OF THE CO2 TENDENCY

(30) Priorité: 11.01.2018 FR 1850225
(43) Date de publication de la demande: 17.07.2019
(73) Titulaire: Air Liquide Medical Systems, 92160 Antony (FR)
(72) Inventeur: RICHARD, Jean-Christophe, 92160 ANTONY (FR); RIGOLLOT, Marceau, 92160 ANTONY (FR); BADAT, Bilal, 92160 ANTONY (FR)
(74) Mandataire: Air Liquide

(56) Documents cités:
- WO-A1-2012/114286
- WO-A1-2012/162048
- US-A1- 2015 328 417

## Description

L'invention porte sur un appareil d'assistance respiratoire, c'est-à-dire un ventilateur médical, connecté à un patient recevant une réanimation cardio-pulmonaire (RCP), c'est-à-dire un patient en arrêt cardiaque soumis à un massage cardiaque avec alternance de compressions décompressions thoraciques, avec affichage d'au moins une valeur moyenne de teneur en CO₂ obtenue, sur une fenêtre de temps donnée, à partir d'une pluralité de valeurs maximales de teneur en CO₂ successives.

Les appareils médicaux de ventilation mécanique, encore appelés appareil d'assistance respiratoire ou ventilateurs médicaux, sont couramment utilisés pour fournir du gaz respiratoire, par exemple de l'air enrichi ou non en oxygène, à certains patients souffrant de troubles respiratoires.

La fourniture du gaz respiratoire au patient est couramment opérée au moyen d'une micro-soufflante motorisée et pilotée, comme notamment décrit par EP-A-3093498, EP-A-2947328, EP-A-2986856, EP-A-2954213 ou EP-A-2102504.

Il est connu de monitorer les composés gazeux présents dans le gaz administré aux patients, en particulier dans les gaz expirés par les patients qui contiennent du CO₂ résultant des échanges gazeux pulmonaires, c'est-à-dire du CO₂ produit par le métabolisme du patient, transporté jusqu'aux poumons grâce à la circulation sanguine puis évacué lors de l'expiration du patient. Ainsi, l'etCO₂ pour End Tidal CO₂ ou CO₂ en fin d'expiration, correspond à la mesure de la fraction de CO₂ en fin d'expiration dans les gaz recueillis lors de l'expiration d'un individu, que son inspiration soit naturelle ou assistée, c'est-à-dire obtenue par ventilation mécanique.

Sous ventilation mécanique, différentes techniques permettent l'analyse par spectrophotométrie de la fraction en CO₂ des gaz expirés. Pour ce faire, le gaz présent dans le circuit expiratoire peut être :
- soit aspiré puis analysé par une cellule d'analyse sur un site éloigné du circuit respiratoire. Cette manière d'opérer est appelée à flux dérivé ou secondaire, i.e. « *sidestream* » en anglais,
- soit analysé près du patient, préférentiellement au niveau d'une pièce Y agencée dans le circuit respiratoire à proximité du patient. Cette manière d'opérer est appelée à flux principal, i.e. « *mainstream »* en anglais.

Pendant une réanimation cardio-pulmonaire ou RCP pratiquée sur une personne en arrêt cardio-respiratoire, le CO₂ alvéolaire, dépend de la quantité de CO₂ généré par le métabolisme cellulaire, du débit cardiaque, et des rapports ventilation/perfusion pulmonaires. En théorie, plus la RCP est efficace, plus le métabolisme cellulaire est conservé et le débit cardiaque généré par les compressions thoraciques est important, plus la quantité de CO2 ramenée vers les poumons est importante. Pour ces raisons, le monitoring de l'EtCO2 est recommandé pour guider la réanimation cardio pulmonaire RCP.

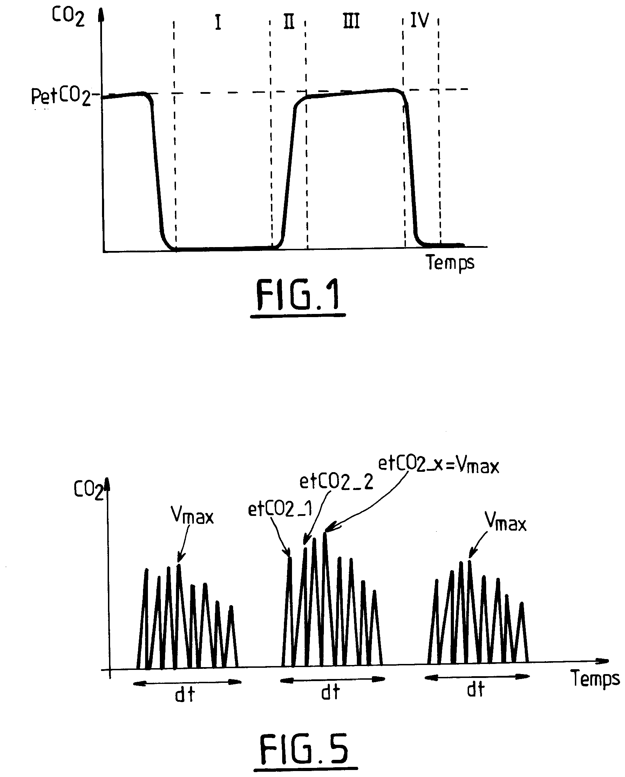

La Figure 1 est un capnogramme qui est une représentation graphique des variations de teneur en CO₂ dans les gaz respiratoires d'un patient au fil du temps (en secondes). Ce type de capnogramme est observé sur les patients ventilés en dehors des situations d'arrêt cardiaque. Comme on le voit, il se divise en quatre phases successives :
- Phase I : elle représente la ligne de base inspiratoire qui doit être stable à zéro.
- Phase II : elle est la partie ascendante du capnogramme et correspond à l'apparition du CO₂ dans les gaz expirés, au début de l'expiration du patient, par vidange des alvéoles les mieux ventilées. En réalité, l'expiration débute un peu avant cette phase car le gaz expiré en début d'expiration est dépourvu de CO₂ puisqu'il n'a pas participé aux échanges gazeux, du fait des espaces-morts instrumental et anatomique. L'augmentation en CO₂ est d'autant plus lente que le poumon est inhomogène et que les alvéoles ont des constantes de temps longues.
- Phase III : elle correspond à la phase de plateau alvéolaire qui correspond au gaz riche en CO₂ en provenance des alvéoles les moins bien ventilées. La valeur maximale de fin de plateau (PetCO2) correspond à la valeur d' etCO₂.
- Phase IV : elle correspond à la diminution de la concentration en CO₂ engendrée par un début d'inspiration spontanée ou assistée (i.e. mécanique).

Toutefois, lors d'une Réanimation Cardio-Pulmonaire (RCP) sur un patient en arrêt cardio-respiratoire, le capnogramme est très différent pour plusieurs raisons, notamment:
- les compressions thoraciques (CT) génèrent des déplacements de petits volumes de gaz. Ces volumes, proches de l'espace mort instrumentale et anatomique, perturbent le capnogramme entre deux cycles ventilatoires par un effet de lavage du CO₂. On observe ainsi souvent des tracés oscillants puisque la valeur maximale de CO₂ sur chaque compression thoracique ne cesse de varier.
- le comportement dynamique d'ouverture et de fermeture des petites voies aériennes lors de la RCP a été récemment rapporté lors de la RCP. Ce phénomène compromet les mouvements de gaz expirés et donc l'interprétation des concentrations de CO₂ lors de la RCP.

On comprend donc que l'etCO₂ tel qu'il est mesuré actuellement, c'est-à-dire lors de chaque compression thoracique ne permet pas d'obtenir une approximation fiable de la teneur en CO₂ alvéolaire.

Or, cette teneur en CO₂ alvéolaire est importante pour le personnel soignant car elle constitue un reflet de la qualité de la RCP et d'une possible reprise d'activité cardiaque spontanée (RACS).

En effet, pendant la RCP, la valeur de concentration en CO₂ ou la tendance en CO₂ est utilisée par le secouriste pratiquant le massage cardiaque, i.e. médecin ou tout autre personnel soignant, comme une « image » de la circulation sanguine et donc de l'efficacité du massage cardiaque qu'il pratique, la tendance en CO₂ étant définie comme une représentation graphique de plusieurs valeurs de concentration en CO₂ mesurées successivement sur une fenêtre de temps donnée, par exemple pendant les 30 secondes à 5 minutes venant de s'écouler.

Le problème récurrent qui en résulte est qu'une ou des mesures de la teneur en CO₂ sans prise en compte tout ou partie de ces facteurs, en particulier de l'impact de la ventilation réalisée sur le patient en arrêt cardiaque, rend l'utilisation pour pronostic de cette mesure de CO₂ peu fiable, voire totalement non fiable.

Les solutions actuelles de monitorage de l'etCO₂ sont adaptées aux variations de CO₂ provoquées par la respiration, qu'elle soit mécanique ou spontanée. Les fréquences en jeu sont de l'ordre de 10 à 30 c/min. Les algorithmes et mécanismes mis en œuvre sont adaptés à ces fréquences et à des variations faibles du CO₂ entre deux respirations du patient.

Or, pendant une réanimation cardio-pulmonaire, les fréquences des compressions thoraciques (CTs) en jeu sont de l'ordre de 100 c/min, les volumes de gaz mobilisés faibles et les débits de gaz importants et irréguliers.

Dans ces conditions, la valeur de l'etCO₂ variant à chaque compression thoracique qui est affichée sur l'interface graphique des ventilateurs actuels, est rafraîchie à une fréquence inadéquate puisque les ventilateurs tentent de suivre l'évolution du CO₂ à la fréquence du massage, soit 100 c/min.

En d'autres termes, la valeur de l'etCO₂ ou la tendance en CO₂ affichée par les ventilateurs actuels n'est pas représentative d'une concentration de CO₂ liée au métabolisme du patient car l'origine du gaz analysé n'est pas garantie.

Les documents WO-A-2014/072981, US-A-2016/133160 et US-A-2012/016279 proposent des méthodes de suivi de la teneur en CO₂ dans les gaz expirés par un patient subissant une RCP, dans lesquelles les ventilateurs indiquent au secouriste qu'il doit stopper le massage cardiaque lorsque la teneur en etCO_{z} est supérieure à 30 mm Hg par exemple. WO2012/114286 A1 divulgue les caractéristiques du préambule de la revendication 1.

Le problème qui se pose est dès lors de proposer un appareil d'assistance respiratoire, c'est-à-dire un ventilateur médical amélioré, qui permette d'afficher, pendant une RCP, une valeur de CO₂ fiable, c'est-à-dire qui reflète le plus possible le CO₂ alvéolaire et ses évolutions dans le temps, dans le but de mieux assister le secouriste durant la RCP en lui proposant une information pertinente facilitant le monitorage de la RCP et permettant ou facilitant une détection de RACS par exemple.

La solution de l'invention concerne alors un appareil d'assistance respiratoire, c'est-à-dire un ventilateur médical, pour fournir un gaz respiratoire, tel de l'air, à un patient pendant une réanimation cardio-pulmonaire (RCP) comprenant :
- une source de gaz respiratoire pour fournir un gaz respiratoire audit patient pendant une réanimation cardio-pulmonaire (RCP),
- des moyens de mesure de teneur en CO₂ pour opérer des mesures de concentration en CO₂ produit par ledit patient, et fournir des signaux de mesure de teneur en CO₂ à des moyens de traitement de signal et de pilotage,
- des moyens de traitement de signal et de pilotage configurés pour traiter les signaux de mesure de teneur en CO₂ provenant des moyens de mesure de teneur en CO₂, et
- au moins une interface graphique utilisateur ou IGU,
caractérisé en ce que :
- les moyens de traitement de signal et de pilotage sont configurés pour :
   a) traiter les signaux de mesure de teneur en CO₂ correspondant à des mesures opérées par les moyens de mesure de teneur en CO₂ pendant une période de temps (dt) donnée, pour en extraire une pluralité de valeurs de teneur en CO₂,
   b) sélectionner la valeur maximale (Vmax) de teneur en CO₂ parmi ladite pluralité de valeurs de teneur en CO₂ mesurées pendant ladite période de temps (dt) donnée,
   c) répéter les étapes a) et b) pour obtenir plusieurs valeurs maximales (Vmax) de teneur en CO₂ successives mesurées pendant une fenêtre de temps (Ft) comprenant plusieurs périodes de temps (dt) successives,
   d) calculer au moins une valeur moyenne (Vmoy) de teneur en CO₂ à partir des valeurs maximales (Vmax) de teneur en CO₂ obtenues sur la fenêtre de temps (Ft), et
   e) transmettre ladite au moins une valeur moyenne (Vmoy) de teneur en CO₂ à l'interface graphique utilisateur ou IGU,
- et l'interface graphique utilisateur est configurée pour afficher ladite au moins une valeur moyenne (Vmoy) de teneur en CO₂.

Selon le cas, l'appareil d'assistance respiratoire de l'invention peut comprendre l'une ou plusieurs des caractéristiques techniques suivantes :
- l'IGU est configurée pour afficher au moins une valeur de teneur en CO₂ fournie par les moyens de traitement de signal et de pilotage.
- l'IGU est configurée pour afficher ladite au moins une valeur moyenne (Vmoy) de teneur en CO₂ sous forme d'une valeur numérique ou d'une représentation graphique, de préférence une représentation graphique, par exemple courbe, barre-graphe ou autre.
- l'IGU est configurée pour afficher la valeur moyenne (Vmoy) de teneur en CO₂ la plus récente, c'est-à-dire la dernière valeur calculée sur une fenêtre de temps (Ft) donnée, en particulier une fenêtre de temps (Ft) glissante.
- l'IGU est en outre configurée pour afficher la valeur maximale (Vmax) de teneur en CO₂ la plus récente, c'est-à-dire la dernière valeur maximale (Vmax) de teneur en CO₂ déterminée pendant la dernière période de temps (dt) d'une fenêtre de temps (Ft) donnée incluant plusieurs périodes de temps (dt) successives, en particulier une fenêtre de temps (Ft) glissante.
- le CO₂ produit par le patient. Ce CO₂ est observé lors de l'expiration du patient, c'est-à-dire notamment dans les gaz expirés, ou réinhalé à l'inspiration suivante lorsqu'il s'agit de gaz piégé dans une partie du circuit respiratoire, par exemple entre une pièce de jonction agencée en amont de l'interface respiratoire, telle une pièce Y, et le capteur de CO₂.
- selon un mode de réalisation, l'IGU est configurée pour afficher au moins une partie des valeurs moyennes (Vmoy) successives de teneur en CO₂ calculées sous forme d'une courbe formée d'une succession de symboles graphiques, chaque symbole graphique correspondant à une valeur moyenne (Vmoy) de teneur en CO₂, en particulier une courbe de tendance.
- chaque valeur moyenne (Vmoy) de teneur en CO₂ est affichée par l'IGU sous forme d'un symbole graphique de type point, croix ou tout autre symbole.
- selon un autre mode de réalisation, l'IGU est configurée pour afficher au moins une partie des valeurs moyennes (Vmoy) successives de teneur en CO₂ calculées sous forme d'un barre-graphe comprenant plusieurs barres, chaque barre dudit barre-graphe correspondant à une valeur moyenne (Vmoy) de teneur en CO₂.
- préférentiellement, l'affichage graphique, notamment de la courbe de tendance ou autre, représentant les variations de la teneur moyenne (Vmoy) en CO₂ est rafraîchi, c'est-à-dire mis à jour, après un intervalle de temps régulier et cyclique, par exemple après quelques secondes.
- les moyens de traitement de signal et de pilotage sont configurés pour répéter les étapes a) à e) de manière à obtenir plusieurs valeurs moyennes (Vmoy) de teneur en CO₂ successives calculées à partir de valeurs maximales (Vmax) de teneur en CO₂ obtenues sur des fenêtres de temps (Ft) successives, en particulier une fenêtre de temps glissante.
- les fenêtres de temps (Ft) successives sont avantageusement une fenêtre de temps glissante.
- la fenêtre de temps (Ft) est comprise entre 20 secondes et 10 minutes, de préférence entre 30 secondes et 5 minutes, de préférence d'au moins 1 minute.
- l'IGU est configurée pour afficher une courbe de tendance formée d'une succession de symboles graphiques, chaque succession de symboles graphiques correspondant à une valeur moyenne (Vmoy) de teneur en CO₂. En d'autres termes, l'IGU affiche sur un graphique temporel représentant une représentation graphique de chaque valeur moyenne (Vmoy) de teneur en CO₂, à savoir un symbole graphique de type point ou croix par exemple, en fonction du temps (en secondes ou minutes). Cet affichage se fait sur une fenêtre de temps glissante allant de 30 sec à 5 minutes par exemple, en particulier de 1 à 3 minutes.
- la source de gaz respiratoire est une source d'air, en particulier une micro-soufflante motorisée, encore appelée turbine ou compresseur.
- les moyens de traitement de signal et de pilotage comprennent au moins une carte électronique.
- les moyens de traitement de signal et de pilotage comprennent au moins un microprocesseur, de préférence un microcontrôleur.
- le microprocesseur met en œuvre au moins un algorithme.
- les moyens de mesure de teneur en CO₂ sont préférentiellement agencés sur le flux principal de gaz, i.e. en « mainstream ».
- alternativement, les moyens de mesure de teneur en CO₂ sont agencés dans le ventilateur, i.e. en « sidestream », le ou les échantillons de gaz étant prélevés dans le flux principal, puis analysés pour en déterminer la teneur en CO₂.
- la source de gaz respiratoire est en communication fluidique avec un conduit de gaz servant à véhiculer le gaz respiratoire vers le patient, i.e. jusqu'à une interface respiratoire.
- le conduit de gaz est en communication fluidique avec une interface respiratoire de manière à alimenter ladite interface avec du gaz provenant de la micro-soufflante.
- les moyens de mesure de teneur en CO₂ sont reliés électriquement aux moyens de traitement de signal et de pilotage.
- les moyens de mesure de teneur en CO₂ sont agencés de manière à opérer des mesures de concentration en CO₂ en aval du conduit de gaz, de préférence au niveau d'une extrémité aval du conduit de gaz.
- les moyens de mesure de teneur en CO₂ sont agencés en amont et à proximité immédiate de l'interface respiratoire, c'est-à-dire proche de la bouche du patient.
- les moyens de mesure de teneur en CO₂ sont agencés sur une pièce de jonction agencée entre l'interface respiratoire et le conduit de gaz.
- les moyens de mesure de teneur en CO₂ sont agencés sur une pièce de jonction agencée entre l'interface respiratoire et une pièce en Y comprenant des passages internes de gaz.
- l'interface respiratoire est une sonde d'intubation endotrachéale, un masque facial ou un masque laryngé, aussi appelé dispositif supra glottique, ou tout dispositif adéquat d'administration de gaz.
- l'interface respiratoire est de préférence une sonde d'intubation endotrachéale, couramment appelée 'sonde trachéale'.
- selon un premier mode de réalisation, les moyens de mesure de teneur en CO₂ sont agencés sur une pièce de jonction agencée en amont de l'interface respiratoire, de préférence entre l'interface respiratoire et l'extrémité aval du conduit de gaz, en particulier entre l'interface respiratoire et une pièce en Y comprenant des passages internes de gaz.
- de préférence, les moyens de mesure de teneur en CO₂ sont agencés sur une pièce de jonction comprenant un passage interne de gaz.
- selon un deuxième mode de réalisation, les moyens de mesure de teneur en CO₂ sont agencés dans l'appareil, c'est-à-dire dans la carcasse de l'appareil, en étant reliés, via un conduit de prélèvement de gaz ou analogue, à un site de prélèvement de gaz situé en amont et à proximité immédiate de l'interface respiratoire.
- en particulier, les moyens de mesure de teneur en CO₂ sont reliés fluidiquement à un site de prélèvement de gaz porté par une pièce de jonction, en particulier agencée entre l'interface respiratoire et le conduit de gaz, typiquement entre l'interface respiratoire et une extrémité aval dudit conduit de gaz.
- la pièce de jonction est raccordée fluidiquement entre la pièce de raccordement intermédiaire, c'est-à-dire une pièce en Y, et l'interface respiratoire.
- il comprend un circuit patient comprenant une branche inspiratoire permettant d'acheminer du gaz vers le patient et une branche expiratoire permettant d'évacuer le gaz expiré par le patient.
- la branche inspiratoire, la branche expiratoire et l'interface respiratoire sont connectées mécaniquement et/ou fluidiquement, directement ou indirectement, à une pièce de raccordement intermédiaire, notamment une pièce en Y.
- le conduit de gaz forme tout ou partie de la branche inspiratoire du circuit de gaz.
- la branche expiratoire communique fluidiquement avec l'atmosphère pour évacuer le gaz expiré par le patient, en particulier un gaz riche en CO₂.
- la branche inspiratoire et/ou la branche expiratoire comprennent des tuyaux flexibles.
- de préférence, tout ou partie du conduit de gaz formant tout ou partie de la branche inspiratoire du circuit de gaz est un tuyau flexible.
- les moyens de mesure de teneur en CO₂ sont agencés de manière à opérer des mesures de concentration en CO₂ dans ou à la sortie de la branche inspiratoire du circuit de gaz.
- les moyens de traitement de signal et de pilotage sont configurés pour commander la source de gaz respiratoire et délivrer le gaz respiratoire selon des cycles ventilatoires successifs, en particulier des cycles ventilatoires comprenant deux niveaux de pression.
- la période de temps donnée (dt) est de plusieurs secondes.
- chaque cycle ventilatoire comprend une phase BP (D_{BP}) pendant laquelle le gaz est délivré par la micro-soufflante à une pression dite basse ou basse pression (BP), et une phase HP (D_{HP}) pendant laquelle le gaz est délivré par la micro-soufflante à une pression dite haute ou haute pression (HP), avec HP > BP.
- la micro-soufflante est pilotée pour fournir du gaz à une pression basse (BP) comprise entre 0 et 20 cm d'eau, de préférence entre 0 et 15 cm d'eau, préférentiellement encore 0 et 10 cm d'eau.
- la micro-soufflante est pilotée pour fournir du gaz à une pression haute (HP) comprise entre 5 et 60 cm d'eau, de préférence entre 5 et 45 cm d'eau, préférentiellement encore 5 et 30 cm d'eau (avec HP > BP).
- la phase BP a une durée supérieure à la phase HP.
- la phase BP a une durée comprise entre 2 et 10 secondes, typiquement de l'ordre de 3 à 6 secondes.
- la phase HP a une durée comprise entre 0,5 et 3 secondes, typiquement de l'ordre de 1 à 2 secondes.
- la période de temps donnée (dt) est de plusieurs secondes.
- la période de temps (dt) est comprise entre 2 et 10 secondes, typiquement de l'ordre de 3 à 6 secondes.
- la période de temps (dt) correspond à la durée de la phase BP de chaque cycle ventilatoire.
- la durée totale d'un cycle ventilatoire est comprise entre 3 et 12 secondes.
- la période de temps (dt) donnée englobe plusieurs durées de compressions thoraciques et de relâchements successifs, typiquement entre 5 et 20 compressions thoraciques.
- les moyens de mesure de teneur en CO₂ sont configurés pour opérer des mesures en continu.
- les moyens de mesure de teneur en CO₂ comprennent un capteur de CO₂.
- les moyens de mesure de teneur en CO₂ comprennent un capnomètre en tant que capteur de CO₂.
- les moyens de mesure de teneur en CO₂ comprennent un capteur de CO₂ dont la prise de mesure est en communication fluidique avec l'intérieur ou lumen de la pièce de jonction agencée en amont de l'interface respiratoire.
- il comprend des moyens de mémorisation coopérant avec les moyens de traitement de signal et de pilotage pour mémoriser la pluralité de valeurs de teneur en CO₂ mesurées pendant la période de temps donnée.
- il comprend des moyens de mémorisation coopérant avec les moyens de traitement de signal et de pilotage pour mémoriser des valeurs maximales (Vmax) et/ou moyennes (Vmoy) de teneur en CO₂.
- les moyens de mémorisation comprennent une mémoire flash ou de type disque dur.
- il comprend en outre des moyens de mesure de débit gazeux configurés pour opérer au moins une mesure, de préférence en continu, du débit du gaz expiré et du débit de gaz inspiré par le patient. Le débit permet le monitorage et la surveillance des compressions thoraciques, ainsi que le calcul, le monitorage et la surveillance des volumes de gaz délivrés et expirés (ventilateur et CTs).
- les moyens de mesure de débit gazeux comprennent un capteur de débit.
- l'interface graphique utilisateur (IGU) comprend un écran digital, de préférence un écran tactile.
- l'écran comprend plusieurs touches tactiles activant différentes fonctions et/ou plusieurs zones ou fenêtres d'affichage.
- l'écran est du type à affichage en couleurs.
- il comprend une source de courant électrique, par exemple une batterie ou analogue, de préférence une batterie rechargeable.
- il comprend des moyens d'alarme configurés pour se déclencher lorsque la valeur maximale (Vmax) ou moyenne (Vmoy) de teneur en CO₂ excède une valeur-seuil.
- les moyens d'alarme comprennent une alarme sonore ou visuelle, ou les deux.
- les moyens d'alarme sont programmés pour se déclencher lorsque la valeur maximale (Vmax) de CO₂ mesurée, à un instant t, est telle que : [VmaxCO₂] > n x [MoyCO₂], où:
   - n est compris entre 1,20 et 2, de préférence entre 1,25 et 1,7, par exemple de l'ordre de 1,5,
   - [VmaxCO₂] est la valeur maximale de teneur en CO₂ mesurée pendant une durée dt donnée, par exemple sur une durée dt comprise entre 2 et 10 secondes,
   - [MoyCO₂] est la valeur moyenne des valeurs maximales de teneur en CO₂ [VmaxCO₂] déterminées pour plusieurs durées dt successives comprises dans une fenêtre de temps (FT) donnée (FT> x.dt avec x ≥ 2), par exemple une période de 30 sec à 5 minutes, ou plus.
- il comprend une carcasse rigide comprenant la source de gaz respiratoire, les moyens de traitement de signal et de pilotage, la source de courant électrique et les moyens de mémorisation.
- l'IGU est agencée, notamment encastrée, dans l'une des parois formant la carcasse du ventilateur.
- la carcasse rigide est formée en tout ou en partie de polymère.
- les moyens de mesure de teneur en CO₂ sont configurés pour opérer des mesures de concentration en CO₂ successives sur des périodes de temps (dt) successives, c'est-à-dire des périodes de temps (dt) espacées les unes des autres.
- les moyens de mesure de teneur en CO₂ sont configurés pour opérer des mesures de concentration en CO₂ successives sur des périodes de temps (dt) successives pendant des cycles ventilatoires successifs, en particulier pendant les phases BP de cycles ventilatoires successifs.

L'invention concerne aussi un procédé (i.e., une méthode) de suivi d'une réanimation cardio-pulmonaire (RCP) opéré sur un patient en arrêt cardiaque, dans lequel :
- on met en œuvre un appareil d'assistance respiratoire comprenant une source de gaz respiratoire, telle une micro-soufflante, pour fournir un gaz respiratoire à un patient pendant une réanimation cardio-pulmonaire (RCP),
- on opère des mesures de concentration en CO₂ produit par ledit patient, par exemple au moyen d'un capnomètre,
- on traite les signaux de mesure de teneur en CO₂, par exemple au moyen de moyens de traitement de signal et de pilotage, telle un microprocesseur,
- on détermine une pluralité de valeurs de teneur en CO₂ mesurées pendant une période de temps (dt) donnée,
- on sélectionne la valeur maximale (Vmax) de teneur en CO₂ parmi la pluralité de valeurs de teneur en CO₂,
- on répète les étapes précédentes pour obtenir plusieurs valeurs maximales (Vmax) de teneur en CO₂ successives mesurées sur une fenêtre de temps (Ft) comprenant plusieurs périodes de temps (dt) successives,
- on calcule au moins une valeur moyenne (Vmoy) de teneur en CO₂ à partir des valeurs maximales (Vmax) de teneur en CO₂ obtenues sur la fenêtre de temps (Ft), et
- on affiche ladite au moins une valeur moyenne (Vmoy) de teneur en CO₂ sur une IGU.

L'invention va maintenant être mieux comprise grâce à la description détaillée suivante, faite à titre illustratif mais non limitatif, en référence aux figures annexées parmi lesquelles :
- la Figure 1 est une représentation graphique des variations de teneur en CO₂ dans les gaz respiratoires d'un patient normal,
- la Figure 2 schématise un cycle ventilatoire à deux niveaux de pression pouvant être mis en œuvre par l'appareil de la Figure 6 pour ventiler un patient en arrêt cardio-pulmonaire au cours d'une RCP,
- la Figure 3 illustre les variations de pression au niveau pulmonaire chez un patient en arrêt cardio-pulmonaire au cours d'une RCP,
- la Figure 4 schématise la quantité de CO₂ mesurée par le capnomètre de l'appareil de la Figure 6 pendant une RCP, et au moment et après retour à une activité cardiaque spontanée (RACS),
- la Figure 5 schématise les pics de teneur en CO₂ pendant les cycles ventilatoires mis en œuvre pendant une RCP,
- la Figure 6 est un schéma d'un mode de réalisation d'un appareil d'assistance respiratoire pour RCP selon l'invention, et
- la Figure 7 schématise les mesures et les intervalles de temps utilisés pour le calcul et l'affichage de la tendance en CO₂.

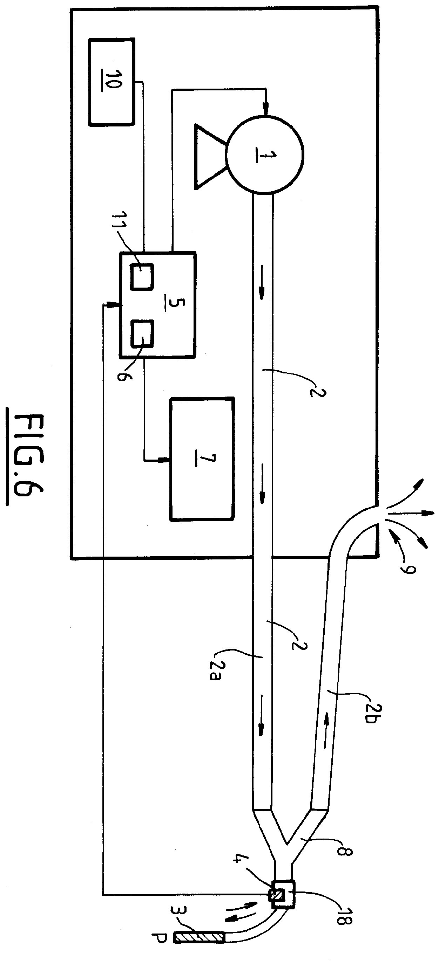

La Figure 6 est une représentation schématique d'un mode de réalisation d'un appareil d'assistance respiratoire ou ventilateur médical selon l'invention servant à fournir un gaz respiratoire, typiquement de l'air ou de l'air enrichi en oxygène, à un patient P pendant une réanimation cardio-pulmonaire (RCP), c'est-à-dire à une personne en arrêt cardiaque sur laquelle un secouriste pratique un massage cardiaque, avec alternance de compressions thoraciques (CT) et de relâchements (Re), c'est-à-dire de non-compressions thoraciques.

L'appareil comprend une source 1 de gaz respiratoire, telle une micro-soufflante motorisée, qui est en communication fluidique avec un conduit de gaz 2 de la branche inspiratoire 2a du circuit patient 2a, 2b pour fournir le gaz respiratoire audit patient P pendant la RCP.

La source 1 de gaz respiratoire est commandée, c'est-à-dire pilotée, par des moyens de traitement de signal et de pilotage 5, notamment une carte électronique à microprocesseur 6 ou analogue. Les moyens de traitement de signal et de pilotage 5 pilotent la source 1 de gaz respiratoire de manière à ce qu'elle délivre le gaz selon un (ou plusieurs) mode de ventilation prédéfinis.

De préférence elle permet de piloter la source de gaz 1 pour délivrer le gaz selon un mode ventilatoire « normal » correspondant à une ventilation d'un patient qui n'est pas en arrêt cardiaque et un mode ventilatoire « RCP » correspondant à une ventilation d'un patient qui est en arrêt cardiaque et sur lequel un secouriste entame ou pratique une RCP.

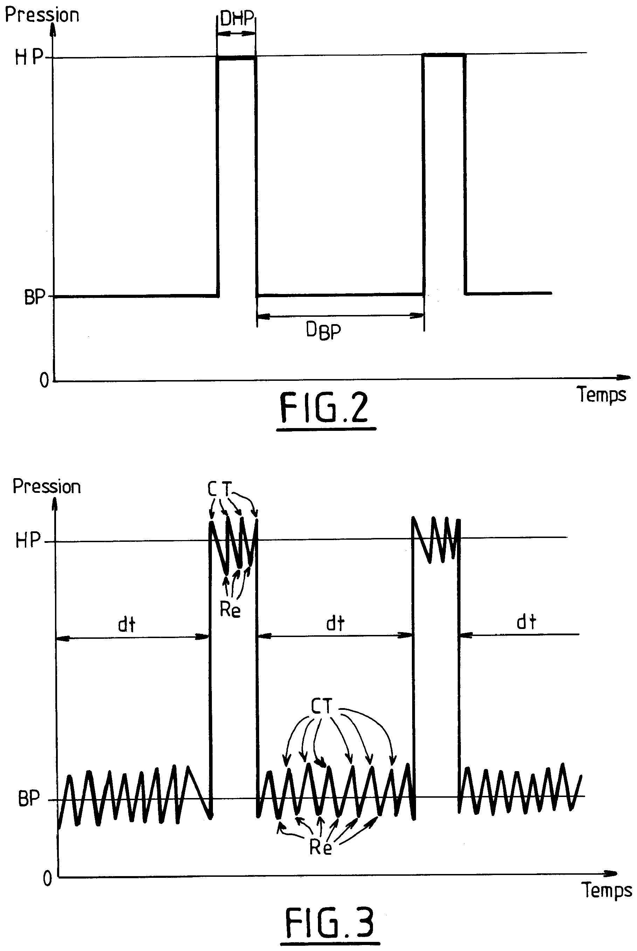

Par exemple, selon un mode de ventilation dédié à la RCP, la source 1 de gaz respiratoire est pilotée pour fournir le gaz respiratoire, typiquement de l'air, selon un cycle ventilatoire comportant plusieurs niveaux de pression ou de type « BiPAP », comme illustré en Figure 2, en particulier 2 niveaux de pression comprenant un niveau de pression basse, par exemple une pression basse (BP) comprise entre environ 0 cm H₂O et 15 cm H₂O, et un niveau de pression haute, par exemple une pression haute (HP) comprise entre environ 7 cm H₂O et 40 cm H₂O.

Le gaz est délivré alternativement entre ces deux niveaux de pression (BP, HP), comme illustré sur la Figure 2, pendant toute la réalisation de la RCP par le secouriste, c'est-à-dire pendant que le secouriste opère les CT et relâchements. La durée (D_{BP}) de fourniture du gaz à pression basse (BP) par la micro-soufflante 1 est comprise entre 2 et 10 secondes, typiquement de l'ordre de 3 à 6 secondes, alors que la durée (D_{HP}) de fourniture du gaz à pression haute (HP) est inférieure à 3 secondes, par exemple de l'ordre de 0,5 à 1,5 secondes.

Les compressions thoraciques (CTs) et relâchements (Re) résultant du massage cardiaque vont eux-mêmes engendrer des variations de pression au niveau des poumons du patient qui vont aller augmenter ou diminuer la pression fournie par la microsoufflante 1 et il en résulte au niveau des poumons du patient, une courbe de pression comme illustré en Figure 3 où les pics de pression au niveau des plateaux hauts (i.e. à PH) et bas (i.e. à PB) reflètent les compressions thoraciques (CT) avec augmentation de pression puisque le thorax s'affaisse sous la pression de la CT opérée par le secouriste, et les relâchements (Re) avec basse de pression puisque le thorax remonte en l'absence de CT.

Comme on le voit sur les Figures 2 et 3, la période de temps (dt) donnée, pendant laquelle la pluralité de valeurs de teneur en CO₂ est mesurée et la valeur maximale de teneur (Vmax) en CO₂ est extraite correspond à la durée (D_{BP}) de fourniture du gaz à pression basse (BP), soit entre 2 et 10 secondes, typiquement entre 3 et 6 secondes.

Le gaz fourni par la micro-soufflante 1 est acheminé par le conduit de gaz 2 qui forme tout ou partie de la branche inspiratoire 2a du circuit patient 2a, 2b. Le gaz respiratoire, en général de l'air, est fourni au patient via une interface de distribution de gaz, par exemple ici une sonde 3 d'intubation endotrachéale, plus simplement appelée 'sonde trachéale'. Toutefois, d'autres interfaces sont utilisables, notamment un masque facial ou un masque laryngé.

Le conduit de gaz 2 de la branche inspiratoire 2a est en communication fluidique avec la sonde trachéale 3 de manière à lui fournir le gaz, tel de l'air, provenant de la source de gaz respiratoire 1. Le conduit de gaz 2 vient en fait se raccorder à la sonde trachéale 3 par l'intermédiaire d'une pièce de raccordement intermédiaire, typiquement une pièce 8 en Y comprenant des passages internes pour le gaz. Cette pièce de raccordement intermédiaire 8 en Y comprend des passages de gaz internes.

La pièce 8 en Y est également raccordée à la branche expiratoire 2b du circuit patient 2a, 2b de manière à pouvoir recueillir et convoyer les gaz riches en CO₂ expirés par le patient P et les évacuer vers l'atmosphère (en 9).

Il est également prévu des moyens de mesure de teneur en CO₂ 4, appelés capteur de CO₂ ou plus simplement capnomètre, conçus pour opérer des mesures de concentration en CO₂ dans le gaz expiré par le patient P, et fournir les signaux de mesure de teneur en CO₂ correspondants aux moyens de traitement de signal et de pilotage 5 où ces signaux de mesure peuvent être traités par un (ou des) algorithme de calcul ou analogue.

Dans le mode de réalisation de la Figure 6, le capteur de CO₂ est agencé près de la bouche du patient P en configuration « flux principal » (*mainstream*)*,* c'est-à-dire en amont et à proximité immédiate de l'interface respiratoire 3, préférentiellement entre la pièce de raccordement intermédiaire 8, i.e. la pièce en Y, et l'interface respiratoire 3, i.e. la sonde trachéale, par exemple sur une pièce de jonction 18 (cf. Fig. 6).

Selon un autre mode de réalisation (non montré), le capteur de CO₂ peut être agencé en configuration « flux dérivé » (*sidestream*)*.* Dans ce cas, le capteur de CO₂ 4 se situe dans la carcasse de l'appareil d'assistance respiratoire et est relié, via une ligne de prélèvement de gaz, telle une tubulure ou analogue, à un site de prélèvement de gaz situé en amont et à proximité immédiate de l'interface respiratoire 3, par exemple sur la pièce de jonction 18. Cette ligne de prélèvement de gaz communique fluidiquement avec le lumen de la pièce de jonction 18 de sorte de pouvoir y prélever du gaz et l'acheminer ensuite jusqu'au capteur de CO₂ situé dans la carcasse de l'appareil.

Dans tous les cas, la pièce de jonction 18 comprend un passage interne de gaz permettant au gaz de la traverser.

Préférentiellement, le capteur de CO₂ opère des mesures en continu de la concentration en CO₂ dans le gaz circulant au travers de la pièce de jonction 18, lequel gaz est enrichi en CO₂ lors de son passage dans les poumons du patient P où s'effectue des échanges gazeux.

Les signaux de mesure de teneur en CO₂ sont ensuite transmis par le capteur de CO₂, par liaison électrique ou analogues, notamment filaire ou autre, aux moyens de traitement de signal et de pilotage 5.

En effet, le monitorage de la teneur en CO₂, en particulier de l'etCO₂ qui reflète indirectement la teneur en CO₂ alvéolaire, est d'une grande importance pendant la RCP, notamment pour détecter un RACS. En effet, un retour d'activité cardiaque spontanée (RACS), donc une augmentation significative du débit cardiaque, engendre une augmentation rapide de la quantité de CO₂ amenée par le sang aux poumons et transférée à travers la membrane alvéolo-capillaire, ce CO₂ se retrouvant ensuite dans le flux gazeux expiré par le patient.

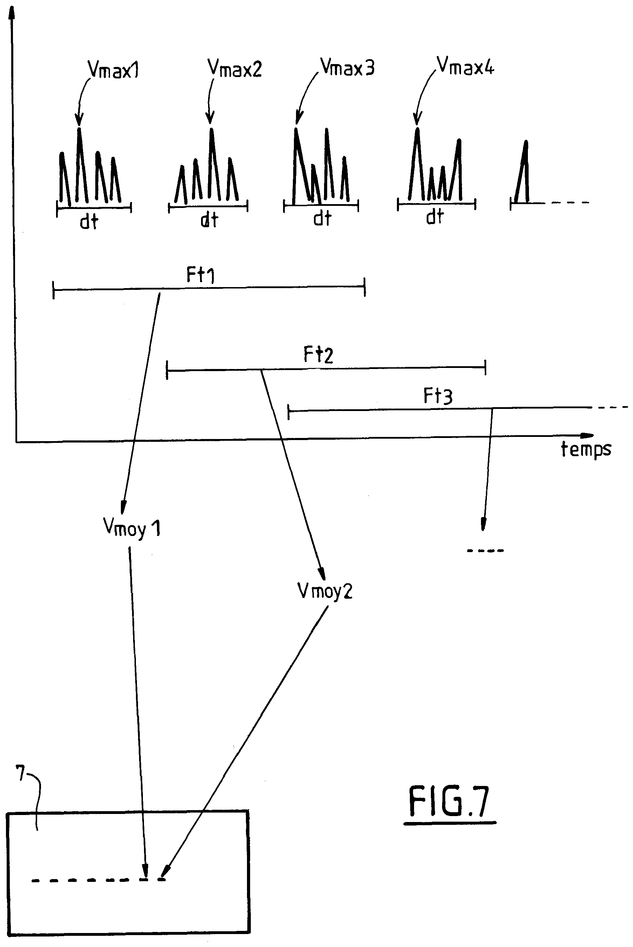

De là, selon la présente invention et comme illustré en Figure 7, les moyens de traitement de signal et de pilotage 5 sont configurés, en particulier le microprocesseur 6, pour :
a) traiter les signaux de mesure de teneur en CO₂ correspondant à des mesures opérées par les moyens de mesure de teneur en CO₂ 4, typiquement un capnomètre, pendant une période de temps (dt) donnée, par exemple entre 1 et 7 secondes, et en extraire une pluralité de valeurs de teneur en CO₂. Pendant la période de temps (dt) considérée, le patient est soumis à un massage cardiaque avec succession de CTs et de relâchements, ce qui engendre des entrées et sorties de gaz des poumons, donc des variations de teneurs en CO₂ dans le flux gazeux expiré, c'est-à-dire sortant des poumons sous l'effet des CTs, notamment en fonction de la force appliquée par le secouriste qui n'est pas égale d'une contraction à l'autre, comme illustré sur les Figures 3 et 5 par exemple.
b) sélectionner la valeur maximale (Vmax) de teneur en CO₂ parmi ladite pluralité de valeurs de teneur en CO₂ mesurées pendant ladite période de temps (dt) donnée. En d'autres termes, parmi les différentes teneurs en CO₂ mesurées pendant la période de temps dt, on sélectionne uniquement la plus élevée qui est la plus représentative de la teneur en CO₂, i.e. en EtCO₂, pendant la période de temps (dt) considérée. Pour ce faire, les moyens de traitement de signal et de pilotage 5 mémorisent puis comparent les valeurs de CO₂ mesurées entre elles pour ne retenir que la plus élevée.
c) répéter les étapes a) et b) pour obtenir plusieurs valeurs maximales (Vmax) de teneur en CO₂ successives mesurées pendant une fenêtre de temps (Ft) plus longue, par exemple entre 30 secondes et 5 minutes, comprenant plusieurs périodes de temps (dt) successives. En d'autres termes, les moyens de traitement de signal et de pilotage 5 opèrent des mesures pendant plusieurs périodes (dt) successives et pour chacune d'elles, sélectionnent la valeur de teneur en CO₂ maximale sur chacune des périodes désirées obtenues pendant la fenêtre de temps longue incluant lesdites périodes (dt) successives. Toutes ces valeurs de teneur en CO₂ maximale sont mémorisées par les moyens de mémorisation 11.
d) calculer au moins une valeur moyenne (Vmoy) de teneur en CO₂ à partir des valeurs maximales (Vmax) de teneur en CO₂ obtenues sur la fenêtre de temps (Ft). Les valeurs maximales (Vmax) de teneur en CO₂ qui ont été mémorisées sur toute la fenêtre de temps (Ft) longue sont retrouvées au sein des moyens de mémorisation 11, puis une valeur moyenne de teneur en CO₂ est calculée à partir de celles-ci pour la la fenêtre de temps (Ft) considérée.
e) transmettre ladite au moins une valeur moyenne (Vmoy) de teneur en CO₂ à l'IGU 7, qui affiche alors cette valeur moyenne (Vmoy) de teneur en CO₂ sous forme d'une valeur numérique ou d'une représentation graphique, avantageusement sous forme d'une représentation graphique, à savoir un symbole graphique, par exemple un point, une croix ou tout autre symbole, qui s'affiche sur un graphique temporel représentant la représentation graphique de la valeur moyenne (Vmoy) de teneur en CO₂ en fonction du temps.
f) les étapes a) à e) sont répétées autant de fois que nécessaire sur des périodes de temps (dt) successives et sur une fenêtre de temps (FT) glissante de durée comprise entre typiquement 1 et 5 minutes, de manière à obtenir des valeurs moyennes (Vmoy) de teneur en CO₂ au fil du temps permettant de suivre l'évolution de la teneur en CO₂ dans le flux gazeux sortant des poumons du patient pendant le massage cardiaque, en particulier sous l'effet des CTs. Dit autrement, l'IGU 7 affiche par exemple une courbe de tendance formée d'une succession de symboles graphiques. Bien entendu, une autre représentation graphique pourrait être adoptée, comme des barre-graphes ou autres.

Le ventilateur médical de l'invention permet une mesure, avantageusement en continu, de la concentration en CO₂ dans les gaz expirés par le patient P. La mesure est opérée par le capnomètre 4 qui est agencé sur le trajet du gaz, au plus proche de la bouche du patient P, préférentiellement entre la pièce 8 en forme de Y et l'interface respiratoire 3, et les signaux de mesure sont transmis, via des liaisons électriques ou autres, aux moyens de traitement de signal et de pilotage 5.

Cette mesure de la concentration en CO₂ dans les gaz expirés par le patient P permet d'obtenir une pluralité de valeurs maximales (Vmax) de teneur en CO₂ qui sont ensuite traitées par les moyens de traitement de signal et de pilotage 5 pour calculer des valeurs moyennes (Vmoy) de teneur en CO₂ à partir de plusieurs valeurs maximales (Vmax) de teneur en CO₂ successives obtenues sur une fenêtre de temps donnée comprenant plusieurs périodes de temps données successives pendant lesquelles les valeurs maximales (Vmax) de teneur en CO₂ ont été déterminées, de préférence une fenêtre de temps glissante (cf. Fig. 7).

La valeur moyenne (Vmoy) de CO₂ n'est pas obligatoirement mise à jour lors de l'affichage de chaque point, mais peut être rafraîchie et affichée après une durée définie, par exemple quelques secondes.

En effet, comme déjà expliqué, la valeur de concentration en CO₂ qui reflète le plus la teneur en CO₂ alvéolaire, et qui donne dès lors une bonne indication de l'état de la circulation sanguine chez le patient P durant la RCP, est la valeur la plus haute de CO₂, encore appelée valeur de pic maximale, comme illustré en Figure 5 qui schématise l'évolution de la teneur en CO₂ et des mesures de l'etCO2, pendant des durées données (dt), dans le cadre d'une RCP pratiquée sur une personne en arrêt cardiaque.

Plus précisément, durant une RCP, la teneur en CO₂ dans le gaz expiré par le patient du fait du massage cardiaque pratiqué, varie en fonction de la présence ou non de compressions thoraciques (CTs).

Ainsi, pendant l'insufflation d'air par la micro-soufflante 1 du ventilateur, puis lors de la ou des premières compressions suivant cette insufflation, on ne détecte pas de CO₂ dans le flux gazeux acheminé par le conduit 2 jusqu'à la pièce 8 en Y, puis à la sonde trachéale 3 qui distribue ensuite cet air aux poumons du patient P. Après quelques compressions thoraciques (CT) pratiquées par un secouriste, du CO₂ est détectée au niveau de la pièce 8 en Y par le capnomètre 4 puisque les alternances de CT et de relâchements (Re) engendrent des mouvements d'air entrant et sortant des poumons du patient.

De l'air expiré riche en CO₂ se retrouve alors au niveau de la pièce 8 en Y et des mesures de concentrations en CO₂ peuvent être réalisées par le capnomètre 4. Les signaux correspondant sont envoyés aux moyens de traitement de signal et de pilotage 5 où ils sont traités comme expliqués ci-avant.

La valeur maximale Vmax de CO₂ déterminée pendant les durées (dt) données, par exemple des durées de 3 à 7 secondes, est celle qui représente le mieux le CO₂ alvéolaire. En effet, le CO₂ présent au niveau de la pièce 8 en Y est "lavé" petit à petit du fait des compressions thoraciques successives et répétées, et tend à diminuer après avoir atteint cette valeur maximale puisque les CT engendrent aussi l'évacuation vers l'atmosphère (en 9) des gaz riches en CO₂, via la branche expiratoire 2b du circuit patient. Les CT successives génèrent donc différents niveaux de CO₂, la plus représentative étant la valeur de pic maximale, comme illustré en Figure 5 qui schématise l'évolution de la teneur en CO₂ dans le gaz et illustre plusieurs mesures de l'etCO₂ mesurées pendant plusieurs durées dt successives, par exemple des durées de 3 à 6 secondes, pendant la réalisation d'une RCP. On y voit que la teneur en CO₂ du gaz n'est pas constante pendant un intervalle de temps dt donné et qu'il existe donc forcément une valeur maximale (Vmax) de teneur en CO₂ sur chaque intervalle dt, c'est-à-dire la valeur de pic.

Le ventilateur mémorise (en 11) donc toutes les valeurs de pics de CO₂ pendant chaque période de temps dt, typiquement entre 3 et 7 secondes, et détermine la valeur maximale Vmax de teneur en CO₂ parmi la pluralité de pics (EtCO2_{_1}, EtCO2_{_2}, EtCO2_{_3}, ..., EtCO2_x) mesurés sur une période de temps (dt) donnée, comme illustré en Figure 5.

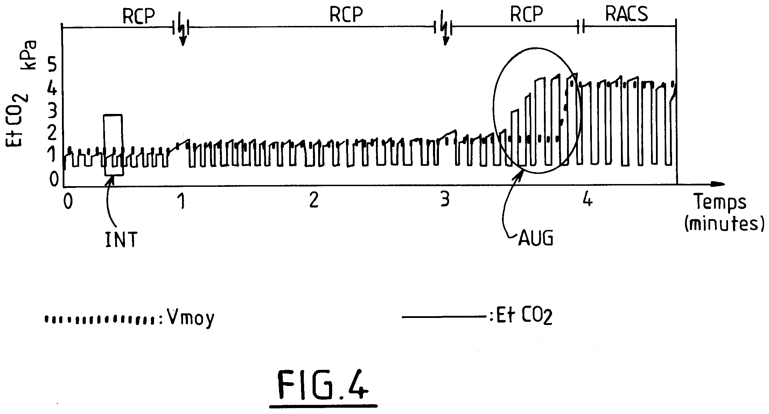

Comme illustré en Figure 7, ces opérations sont répétées au fil du temps sur plusieurs périodes de temps (dt) données successives comprises dans une fenêtre de temps (Ft) plus longue, par exemple une fenêtre de temps (Ft) de 30 sec à 5 min, avantageusement une fenêtre de temps (Ft) glissante, de sorte de pouvoir déterminer et afficher sur l'IGU 7, de préférence en continu, une pluralité de valeurs moyennes de teneur en CO₂ (Vmoy) sous forme d'une représentation graphique, de préférence une courbe de tendance au fil du temps, sur laquelle des symboles graphiques représentent les différentes valeurs moyennes de teneur en CO₂ (Vmoy) en fonction du temps, comme illustré en Figure 4.

Par ailleurs, ces valeurs maximales Vmax de teneur en CO₂ sont traitées par les moyens de traitement de signal et de pilotage 5 de manière à calculer une succession de valeurs moyennes Vmoy de teneur en CO₂, sur une fenêtre de temps donnée, comprenant plusieurs périodes de temps données successives pendant lesquelles lesdites valeurs maximales Vmax de teneur en CO₂ ont été déterminées, de préférence une fenêtre de temps glissante, par exemple une fenêtre de temps comprise entre 30 secondes et 5 minutes.

Les valeurs moyennes Vmoy de teneur en CO₂ ainsi déterminées sont affichées sur l'IGU 7 également sous forme d'une représentation graphique, telle une courbe, un barregraphe ou autre , de préférence sous forme une courbe de tendance sur laquelle les valeurs moyennes (Vmoy) sont représentées par une succession de symboles, tels des points ou analogues (Fig. 4). Sur la Figure 4, la courbe « ...... » représente les valeurs de Vmoy et la courbe « _ » représente les valeurs de Et-CO2.

Les données calculées à partir de ce CO₂, en particulier les valeurs de Vmoy, constituent un indicateur utile au secouriste, lui permettent de piloter la RCP, puisqu'il reflète l'état de la circulation et du métabolisme du patient à partir du moment où le patient est intubé (INT) et qu'une RCP est pratiquée (cf. Fig. 4). En effet, plus la RCP est efficace plus la quantité de CO₂ produite et transférée à travers la membrane alvéolo-capillaire est importante et donc plus la quantité de CO₂ pouvant être détectée au niveau du capnomètre 4 est importante.

De là, en cas de retour à une activité cardiaque spontanée (RACS), la circulation reprend brutalement et donc la quantité de CO₂ alvéolaire augmente parallèlement, ce qui induit une augmentation importante la quantité de CO₂ perçu par le capnomètre 4 d'un facteur souvent supérieur à 2, comme illustré sur la Figure 4. En effet, on voit sur la Figure 4 que l'EtCO₂ est toujours inférieure à 2.5 pendant la RCP, mais qu'elle augmente (AUG) subitement jusqu'à atteindre plus de 5 au moment du RACS, soit après environ 3 à 4 minutes après l'intubation (INT) du patient et le début de la RCP.

Dans le cadre de l'invention, le fait d'afficher sur l'IGU 7, une courbe de tendance basée sur les valeurs moyennes Vmoy déterminée sur une fenêtre de temps (Ft) glissante permet au secouriste de mieux détecter la survenance du RACS puisque la courbe Vmoy fait apparaître une forte augmentation (AUG sur Fig. 4) au moment d'un RACS du fait de la libération accrue de CO₂ par les poumons se retrouvant dans les gaz expirés.

Ainsi, lorsque le secouriste constate une forte élévation (AUG) de la courbe de valeurs moyennes Vmoy en CO₂ sur l'IGU 7, celui-ci peut en conclure que le patient est début de RACS et peut par exemple décider d'analyser le rythme cardiaque et éventuellement de stopper le massage cardiaque.

Le ventilateur permet en outre d'opérer en parallèle une mesure en continu des débits des gaz expirés et inspirés, à l'aide d'un capteur de débit (non montré).

Avantageusement, le ventilateur de l'invention peut aussi inclure des moyens d'alarme conçue et programmée pour avertir le secouriste ou analogue lorsqu'une (ou plusieurs) valeur maximale de CO₂ mesurée excède ou, à l'inverse, devient inférieure une valeur donnée, préfixée ou calculée en continu.

En particulier, on prévoit une alarme sonore et/ou visuelle qui se déclenche lorsque la valeur maximale de CO₂ mesurée, à un instant t, est supérieure à une valeur-seuil, par exemple : [VmaxCO₂] > 1,5 x [MoyCO₂] où:
- [VmaxCO₂] est la valeur maximale de teneur en CO₂ mesurée pendant une durée dt donnée, par exemple sur une durée dt comprise entre 2 et 10 secondes,
- [MoyCO₂] est la valeur moyenne des valeurs maximales de teneur en CO₂ [VmaxCO₂] déterminées pour plusieurs durées dt successives comprises dans une fenêtre de temps (FT) donnée (FT> x.dt avec x ≥ 2), par exemple une période de 30 sec à 5 minutes, ou plus.

De façon analogue, l'alarme peut se déclencher en cas de chute brutale de la concentration en CO₂ sous une valeur minimale donnée qui pourrait être le signe d'un nouvel arrêt cardiaque du patient, d'une hyperventilation ou d'une obstruction du circuit de gaz entre le patient et la machine, par exemple un conduit flexible plié ou écrasé et ne laissant plus passer le gaz.

Une source de courant électrique 10, telle une batterie rechargeable ou analogue, intégrée à la carcasse du ventilateur, alimente, directement ou indirectement, en courant électrique les moyens de traitement de signal et de pilotage 5, la micro-soufflante 1, l'IGU 7 ou tout autre élément de l'appareil, notamment une mémoire 11.

D'une façon générale, l'invention porte sur un ventilateur médical adapté à une utilisation pendant une réanimation cardio-pulmonaire (RCP) comprenant une source de gaz respiratoire 1, telle une micro-soufflante, des moyens de mesure de teneur en CO₂ 4, tel un capnomètre, des moyens de traitement de signal et de pilotage 5 recevant et traitant les signaux de mesure de teneur en CO₂ provenant des moyens de mesure de teneur en CO₂ 4 pour obtenir des valeurs maximales (Vmax) de teneur en CO₂ successives mesurées sur une fenêtre de temps (Ft), et calculer au moins une valeur moyenne (Vmoy) de teneur en CO₂ à partir des valeurs maximales (Vmax) de teneur en CO₂ obtenues sur la fenêtre de temps (Ft), et une IGU 7 configurée pour afficher ladite au moins une valeur moyenne (Vmoy) de teneur en CO₂.

L'appareil d'assistance respiratoire ou ventilateur médical selon la présente invention est particulièrement adapté à une mise en œuvre pendant une réanimation cardio-pulmonaire (RCP) sur une personne (i.e. un patient) en arrêt cardio-pulmonaire, dans le cadre de laquelle un gaz respiratoire, tel de l'air sous pression, est fourni selon un cycle ventilatoire à plusieurs niveaux de pression à ladite personne subissant le massage cardiaque avec alternance de compressions thoraciques et de relâchements. Pour faciliter son transport par les secouristes, par exemple un médecin, un infirmier, un pompier ou analogue, le ventilateur de l'invention est préférentiellement agencé dans un sac de transport.

## Revendications

1. Appareil d'assistance respiratoire pour fournir un gaz respiratoire à un patient pendant une réanimation cardio-pulmonaire (RCP) comprenant :
- une source (1) de gaz respiratoire pour fournir un gaz respiratoire audit patient pendant une réanimation cardio-pulmonaire (RCP),
- des moyens de mesure de teneur en CO₂ (4) pour opérer des mesures de concentration en CO₂ produit par le patient, et fournir des signaux de mesure de teneur en CO₂ à des moyens de traitement de signal et de pilotage (5),
- des moyens de traitement de signal et de pilotage (5) configurés pour traiter les signaux de mesure de teneur en CO₂ provenant des moyens de mesure de teneur en CO₂ (4), et
- au moins une interface graphique utilisateur (7),
**caractérisé en ce que** :
- les moyens de traitement de signal et de pilotage (5) sont configurés pour :
a) traiter les signaux de mesure de teneur en CO₂ correspondant à des mesures opérées par les moyens de mesure de teneur en CO₂ (4) pendant une période de temps (dt) donnée, et en extraire une pluralité de valeurs de pics de teneur en CO₂ (EtCO2_{_1}, EtCO2_{_2}, EtCO2_{_3}, ..., EtCO2_x),
b) sélectionner la valeur maximale (Vmax) de teneur en CO₂ parmi ladite pluralité de valeurs de pics de teneur en CO₂ (EtCO2_{_1}, EtCO2_{_2}, EtCO2_{_3}, ..., EtCO2_x) mesurées pendant ladite période de temps (dt) donnée,
c) répéter les étapes a) et b) pour obtenir plusieurs valeurs maximales (Vmax) de teneur en CO₂ successives mesurées sur une fenêtre de temps (Ft) comprenant plusieurs périodes de temps (dt) successives,
d) calculer au moins une valeur moyenne (Vmoy) de teneur en CO₂ à partir des valeurs maximales (Vmax) de teneur en CO₂ obtenues sur la fenêtre de temps (Ft), et
e) transmettre ladite au moins une valeur moyenne (Vmoy) de teneur en CO₂ à l'interface graphique utilisateur (7),
- et l'interface graphique utilisateur (7) est configurée pour afficher ladite au moins une valeur moyenne (Vmoy) de teneur en CO₂.

2. Appareil selon la revendication précédente, **caractérisé en ce que** les moyens de traitement de signal et de pilotage (5) sont configurés pour répéter les étapes a) à e) de manière à obtenir plusieurs valeurs moyennes (Vmoy) de teneur en CO₂ successives calculées à partir de valeurs maximales (Vmax) de teneur en CO₂ obtenues sur des fenêtres de temps (Ft) successives, de préférence une fenêtre de temps (Ft) glissante.

3. Appareil selon l'une des revendications précédentes, **caractérisé en ce que** la fenêtre de temps (Ft) est comprise entre 20 secondes et 10 minutes, de préférence entre 30 secondes et 5 minutes.

4. Appareil selon l'une des revendications précédentes, **caractérisé en ce que** l'interface graphique utilisateur (7) est configurée pour afficher ladite au moins une valeur moyenne (Vmoy) de teneur en CO₂ sous forme d'une représentation graphique ou d'une valeur numérique, de préférence sous forme d'une représentation graphique.

5. Appareil selon l'une des revendications précédentes, **caractérisé en ce que** l'interface graphique utilisateur (7) est configurée pour afficher au moins une partie des valeurs moyennes (Vmoy) successives de teneur en CO₂ calculées sous forme :
- d'une courbe formée d'une succession de symboles graphiques, chaque symbole graphique correspondant à une valeur moyenne (Vmoy) de teneur en CO₂,
- ou d'un barre-graphe comprenant plusieurs barres, chaque barre dudit barre-graphe correspondant à une valeur moyenne (Vmoy) de teneur en CO₂.

6. Appareil selon l'une des revendications précédentes, **caractérisé en ce que** les moyens de traitement de signal et de pilotage (5) comprennent au moins un microprocesseur.

7. Appareil selon l'une des revendications précédentes, **caractérisé en ce que** les moyens de mesure de teneur en CO₂ (4) comprennent un capnomètre.

8. Appareil selon l'une des revendications précédentes, **caractérisé en ce que** la source (1) de gaz respiratoire est en communication fluidique avec un conduit de gaz (2), le conduit de gaz (2) étant en communication fluidique avec une interface respiratoire (3), de préférence une sonde d'intubation endotrachéale, un masque facial ou un masque laryngé.

9. Appareil selon la revendication 8, **caractérisé en ce que** les moyens de mesure de teneur en CO₂ (4) sont agencés :
- soit en amont et à proximité immédiate (18) de l'interface respiratoire (3),
- soit dans l'appareil en étant reliés à un site de prélèvement de gaz (18) situé en amont et à proximité immédiate de l'interface respiratoire (3).

10. Appareil selon l'une des revendications précédentes, **caractérisé en ce que** la période de temps donnée (dt) est comprise entre 2 et 10 secondes, typiquement de l'ordre de 3 à 6 secondes.

11. Appareil selon l'une des revendications précédentes, **caractérisé en ce que** les moyens de mesure de teneur en CO₂ (4) sont configurés pour opérer des mesures en continu.

12. Appareil selon l'une des revendications précédentes, **caractérisé en ce qu'**il comprend des moyens de mémorisation (8) coopérant avec les moyens de traitement de signal et de pilotage (5) pour mémoriser les valeurs de teneur en CO₂ mesurées pendant chaque période de temps (dt) donnée et les valeurs maximales (Vmax) de teneur en CO₂ calculées pour chaque fenêtre de temps (Ft).

13. Appareil selon l'une des revendications précédentes, **caractérisé en ce que** l'interface graphique utilisateur (IGU) comprend un écran digital, de préférence un écran tactile.

14. Appareil selon l'une des revendications précédentes, **caractérisé en ce que** les moyens de traitement de signal et de pilotage (5) sont configurés pour commander la source (1) de gaz respiratoire et délivrer le gaz respiratoire selon des cycles ventilatoires comprenant deux niveaux de pression, de préférence la source (1) de gaz respiratoire comprend une micro-soufflante motorisée.

## Patentansprüche

1. Beatmungsunterstützungseinrichtung zum Bereitstellen eines Beatmungsgases für einen Patienten während einer Herz-Lungen-Wiederbelebung (RCP), umfassend:
- eine Beatmungsgasquelle (1) zum Bereitstellen eines Beatmungsgases für den Patienten während einer Herz-Lungen-Wiederbelebung (RCP),
- Mittel zum Messen des CO₂-Gehalts (4) zum Vornehmen von Messungen einer Konzentration von vom Patienten erzeugten CO₂, und Bereitstellen von Messsignalen des CO₂-Gehalts für Signalverarbeitungs- und Steuerungsmittel (5),
- Signalverarbeitungs- und Steuerungsmittel (5), die konfiguriert sind, um die Messsignale des CO₂-Gehalts, die aus den Mitteln zum Messen des CO₂-Gehalts (4) stammen, zu verarbeiten, und
- mindestens eine grafische Benutzerschnittstelle (7),
**dadurch gekennzeichnet, dass**:
- die Signalverarbeitungs- und Steuerungsmittel (5) konfiguriert sind zum:
a) Verarbeiten der Messsignale des CO₂-Gehalts entsprechend durch die Mittel zum Messen des CO₂-Gehalts (4) während einer gegebenen Zeitdauer (dt) vorgenommenen Messungen, und daraus Extrahieren einer Vielzahl von Spitzenwerten des CO₂-Gehalts (EtCO2_{_1}, EtCO_{2_2}, EtC02_{_3}, ..., EtCO2_x),
b) Auswählen des maximalen Werts (Vmax) des CO₂-Gehalts aus der Vielzahl von Spitzenwerten des CO₂-Gehalts (EtCO2_{_1}, EtCO2_{_2}, EtCO2_{_3}, ..., EtCO2_x), die während der gegebenen Zeitdauer (dt) gemessen werden,
c) Wiederholen der Schritte a) und b), um mehrere aufeinanderfolgende maximale Werte (Vmax) des CO₂-Gehalts zu erhalten, die über ein Zeitfenster (Ft) gemessen werden, das mehrere aufeinanderfolgende Zeitperioden (dt) umfasst,
d) Berechnen mindestens eines mittleren Wertes (Vmoy) des CO₂-Gehalts aus den über das Zeitfenster (Ft) erhaltenen maximalen Werten (Vmax) des CO₂-Gehalts, und
e) Übertragen des mindestens einen mittleren Werts (Vmoy) des CO₂-Gehalts an die grafische Benutzerschnittstelle (7),
- und die grafische Benutzerschnittstelle (7) konfiguriert ist, um den mindestens einen mittleren Wert (Vmoy) des CO₂-Gehalts anzuzeigen.

2. Einrichtung nach dem vorstehenden Anspruch, **dadurch gekennzeichnet, dass** die Signalverarbeitungs- und Steuerungsmittel (5) konfiguriert sind, um die Schritte a) bis e) derart zu wiederholen, dass mehrere aufeinanderfolgende mittlere Werte (Vmoy) des CO₂-Gehalts erhalten werden, die aus maximalen Werten (Vmax) des CO₂-Gehalts berechnet werden, die über aufeinanderfolgende Zeitfenster (Ft), bevorzugt ein gleitendes Zeitfenster (Ft), erhalten werden.

3. Einrichtung nach einem der vorstehenden Ansprüche, **dadurch gekennzeichnet, dass** das Zeitfenster (Ft) zwischen 20 Sekunden und 10 Minuten, bevorzugt zwischen 30 Sekunden und 5 Minuten, beträgt.

4. Einrichtung nach einem der vorstehenden Ansprüche, **dadurch gekennzeichnet, dass** die grafische Benutzerschnittstelle (7) konfiguriert ist, um den mindestens einen mittleren Wert (Vmoy) des CO₂-Gehalts in Form einer grafischen Darstellung oder eines numerischen Wertes, bevorzugt in Form einer grafischen Darstellung, anzuzeigen.

5. Einrichtung nach einem der vorstehenden Ansprüche, **dadurch gekennzeichnet, dass** die grafische Benutzerschnittstelle (7) konfiguriert ist, um mindestens einen Teil der berechneten aufeinanderfolgenden mittleren Werte (Vmoy) des CO₂-Gehalts anzuzeigen, in Form:
- einer Kurve, die aus einer Folge von grafischen Symbolen besteht, wobei jedes grafische Symbol einem mittleren Wert (Vmoy) des CO₂-Gehalts entspricht,
- oder eines Balkendiagramms, das mehrere Balken umfasst, wobei jeder Balken des Balkendiagramms einem mittleren Wert (Vmoy) des CO₂-Gehalts entspricht.

6. Einrichtung nach einem der vorstehenden Ansprüche, **dadurch gekennzeichnet, dass** die Signalverarbeitungs- und Steuerungsmittel (5) mindestens einen Mikroprozessor umfassen.

7. Einrichtung nach einem der vorstehenden Ansprüche, **dadurch gekennzeichnet, dass** die Mittel zum Messen des CO₂-Gehalts (4) ein Kapnometer umfassen.

8. Einrichtung nach einem der vorstehenden Ansprüche, **dadurch gekennzeichnet, dass** die Beatmungsgasquelle (1) in Fluidverbindung mit einer Gasleitung (2) steht, wobei die Gasleitung (2) in Fluidverbindung mit einer Beatmungsschnittstelle (3), bevorzugt einer endotrachealen Tubus-Sonde, einer Gesichtsmaske oder einer Larynxmaske steht.

9. Einrichtung nach Anspruch 8, **dadurch gekennzeichnet, dass** die Mittel zum Messen des CO₂-Gehalts (4) wie folgt angeordnet sind:
- entweder stromaufwärts und in unmittelbarer Nähe (18) der Beatmungsschnittstelle (3),
- oder in der Einrichtung, indem sie an eine Gasentnahmestelle (18) angeschlossen sind, die sich stromaufwärts und in unmittelbarer Nähe der Beatmungsschnittstelle (3) befindet.

10. Einrichtung nach einem der vorstehenden Ansprüche, **dadurch gekennzeichnet, dass** die gegebene Zeitdauer (dt) zwischen 2 und 10 Sekunden, in der Regel in der Größenordnung von 3 bis 6 Sekunden, beträgt.

11. Einrichtung nach einem der vorstehenden Ansprüche, **dadurch gekennzeichnet, dass** die Mittel zum Messen des CO₂-Gehalts (4) konfiguriert sind, um kontinuierlich Messungen vorzunehmen.

12. Einrichtung nach einem der vorstehenden Ansprüche, **dadurch gekennzeichnet, dass** sie Speichermittel (8) umfasst, die mit den Signalverarbeitungs- und Steuerungsmitteln (5) zusammenwirken, um die während jeder gegebenen Zeitdauer (dt) gemessenen Werte des CO₂-Gehalts und die für jedes Zeitfenster (Ft) berechneten maximalen Werte (Vmax) des CO₂-Gehalts zu speichern.

13. Einrichtung nach einem der vorstehenden Ansprüche, **dadurch gekennzeichnet, dass** die grafische Benutzerschnittstelle (IGU) einen digitalen Bildschirm, bevorzugt einen berührungsempfindlichen Bildschirm, umfasst.

14. Einrichtung nach einem der vorstehenden Ansprüche, **dadurch gekennzeichnet, dass** die Signalverarbeitungs- und Steuerungsmittel (5) konfiguriert sind, um die Beatmungsgasquelle (1) zu regeln und das Beatmungsgas gemäß Beatmungszyklen bereitzustellen, die zwei Druckstufen umfassen, wobei bevorzugt die Beatmungsgasquelle (1) ein motorisiertes Mikrogebläse umfasst.

## Claims

1. Breathing assistance apparatus for supplying a respiratory gas to a patient during a cardiopulmonary resuscitation (CPR) comprising:
- a respiratory gas source (1) for supplying a respiratory gas to said patient during a cardiopulmonary resuscitation (CPR),
- means for measuring CO₂ content (4) for taking measurements of CO₂ concentration produced by the patient, and supplying signals for measuring CO₂ content to signal and control processing means (5),
- signal and control processing means (5) configured for processing the CO₂ content measurement signals coming from the CO₂ content measurement means (4), and
- at least one user graphic interface (7),
**characterised in that**:
- the signal and control processing means (5) are configured for:
a) processing CO₂ content measurement means corresponding to measurements taken by the CO₂ content measurement means (4) for a given time period (dt), and extracting a plurality of CO₂ content peak values from it (EtCO2_{_1}, EtCO_{2_2}, EtCO2_{_3}, ..., EtCO2_x),
b) selecting the maximum value (Vmax) of CO₂ content among said plurality of CO₂ content peak values (EtCO2_₁, EtCO2_₂, EtCO2_₃, ..., EtCO2_x) measured for said given time period (dt),
c) repeating steps a) and b) for obtaining several successive maximum values (Vmax) of CO₂ content measured over a time window (Ft) comprising several successive time periods (dt),
d) calculating at least one average value (Vmoy) of CO₂ content from the maximum values (Vmax) of CO₂ content obtained over the time window (Ft), and
e) transmitting said at least one average value (Vmoy) of CO₂ content to the user graphic interface (7),
- and the user graphic interface (7) is configured for displaying said at least one average value (Vmoy) of CO₂ content.

2. Apparatus according to the preceding claim, **characterised in that** the signal and control processing means (5) are configured for repeating steps a) to e) so as to obtain several successive average values (Vmoy) of CO₂ content calculated from maximum values (Vmax) of CO₂ content obtained over successive time windows (Ft), preferably a sliding time window (Ft).

3. Apparatus according to one of the preceding claims, **characterised in that** the time window (Ft) is comprised between 20 seconds and 10 minutes, preferably between 30 seconds and 5 minutes.

4. Apparatus according to one of the preceding claims, **characterised in that** the user graphic interface (7) is configured for displaying said at least one average value (Vmoy) of CO₂ content in the form of a graphic representation or of a numerical value, preferably in the form of a graphic representation.

5. Apparatus according to one of the preceding claims, **characterised in that** the user graphic interface (7) is configured for displaying at least some of the successive average values (Vmoy) of CO₂ content calculated in the form of:
- a curve formed of a succession of graphic symbols, each graphic symbol corresponding to an average value (Vmoy) of CO₂ content,
- or a bar graph comprising several bars, each bar of said bar graph corresponding to an average value (Vmoy) of CO₂ content.

6. Apparatus according to one of the preceding claims, **characterised in that** the signal and control processing means (5) comprise at least one microprocessor.

7. Apparatus according to one of the preceding claims, **characterised in that** the CO₂ content measurement means (4) comprise a capnometer.

8. Apparatus according to one of the preceding claims, **characterised in that** the respiratory gas source (1) is in fluidic communication with a gas conduit (2), the gas conduit (2) being in fluidic communication with a respiratory interface (3), preferably an endotracheal breathing tube, a facemask or a laryngeal mask.

9. Apparatus according to claim 8, **characterised in that** the CO₂ content measurement means (4) are arranged:
- either upstream and in the immediate proximity (18) of the respiratory interface (3),
- or in the apparatus by being connected to a gas sample site (18) situated upstream and in the immediate proximity of the respiratory interface (3).

10. Apparatus according to one of the preceding claims, **characterised in that** the given time period (dt) is comprised between 2 and 10 seconds, typically of the order of 3 to 6 seconds.

11. Apparatus according to one of the preceding claims, **characterised in that** the CO₂ content measurement means (4) are configured to take measurements continuously.

12. Apparatus according to one of the preceding claims, **characterised in that** it comprises storage means (8) engaging with the signal and control processing means (5) for storing the CO₂ content values measured for each given time period (dt) and the maximum values (Vmax) of CO₂ content calculated for each time window (Ft).

13. Apparatus according to one of the preceding claims, **characterised in that** the user graphic interface (IGU) comprises a digital screen, preferably a touchscreen.

14. Apparatus according to one of the preceding claims, **characterised in that** the signal and control processing means (5) are configured for controlling the respiratory gas source (1) and delivering the respiratory gas according to ventilation cycles comprising two pressure levels, preferably the respiratory gas source (1) comprises a motorised micro fan.
